# EUROPEAN PATENT SPECIFICATION

(11) **EP 1 833 891 B1**
(45) Date of publication and mention of the grant of the patent: **16.06.2010**
(21) Application number: 05822591.3
(22) Date of filing: 26.10.2005
(51) Int. Cl.: C08J 7/12, A61L 29/00, A61M 25/00

(54) **MEDICAL DEVICES AND PROCESSES FOR PREPARING SAME**
MEDIZINISCHE VORRICHTUNGEN UND VERFAHREN ZU IHRER HERSTELLUNG
DISPOSITIFS MÉDICAUX ET LEURS PROCÉDÉS DE PRÉPARATION

(30) Priority: 07.12.2004 US 5734
(43) Date of publication of application: 19.09.2007
(73) Proprietor: Boston Scientific Limited, Christ Church (BB)
(72) Inventor: BURGMEIER, Robert, Plymouth, Minnesota 55447 (US); HORN, Daniel J., Shoreview, Minnesota 55126 (US)
(74) Representative: Altenburg, Udo
(86) International application number: PCT/US2005/038808
(87) International publication number: WO 2006/062611

(56) References cited:
- US-A- 4 404 256
- US-A- 4 536 179
- US-A- 4 902 529
- US-A- 6 156 435
- PATENT ABSTRACTS OF JAPAN vol. 013, no. 290 (C-614), 5 July 1989 (1989-07-05) & JP 01 085665 A (TERUMO CORP), 30 March 1989 (1989-03-30)

## Description

The present invention relates to the field of intraluminal medical devices, such as intraluminal catheters, and in particular intraluminal balloon catheters.

Intraluminal balloon catheters are used for a variety of applications including delivery of medical devices such as stent delivery, for percutaneous transluminal coronary angioplasty (PTCA), cutting balloon catheters for recanalizing and dilating a diseased vessel and facilitating balloon angioplasty procedures, and so forth.

PTCA is a widely used procedure for the treatment of coronary heart disease. In PTCA the balloon catheter is used to restore free flow in a clogged coronary vessel. The catheter is maneuvered through the patient's vasculature and into the patient's coronary anatomy until the balloon is properly positioned across the stenosis to be dilated. This involves a torturous path with very little room inside the vessel. Once properly positioned, the balloon is inflated within the stenotic region of the artery one or more times to a predetermined size to reopen the coronary passageway and increase the blood flow therethrough.

Balloon catheters generally comprise an elongated shaft with an inflatable balloon on the distal end of the shaft. An inflation lumen extending within the shaft is used to deliver inflation fluid to the balloon interior. In over the wire or rapid exchange designs, a guidewire is slidably received within a guidewire lumen extending at least within a distal section of the catheter.

A lubricious coating may be provided on the outer surface of the catheter shaft to facilitate the movement of the catheter within the patient's body lumen. Additionally, a lubricious coating may be provided on an inner surface of the shaft which defines the guidewire lumen, to facilitate the movement of a guidewire therein. The lubricious coatings often comprise silicone or hydrophilic polymeric materials which become lubricious after absorbing water.

One problem which may occur with lubricious coatings having high lubricity, is poor adhesion to the catheter shaft surface. Other challenges include providing high lubricity without a loss of other catheter shaft characteristics such as low profile, strength, flexibility, and ease of manufacture.

One method employed is to extrude a tie layer over the base material, and then a lubricious material over that. This process decreases manufacturing efficiency.

One commonly used low friction surface is polytetrafluoroethylene (PTFE) because of its very low coefficient of friction. However, because of its very low coefficient of friction, it is difficult to wet out the surface and consequently, it can be difficult to adhere other polymers to it, making it difficult to use as a low friction coating. Furthermore, PTFE is very difficult to process.

There remains a need for an improved lubricious surface which provide the catheter shaft with a desirable combination of properties such as good pushability and kink resistance, and a low profile. In this context the documents US 4,536,179, US 6,156,435 and JP 01 085665 A disclose medical devices with different polymer surfaces.

### SUMMARY OF THE INVENTION

In one aspect, the present invention relates to medical devices that have a surface that is modified by having fluorine covalently bonded thereto. In particular embodiments, the portion of the device having the surface which is modified is formed of a polymer material.

More particularly, the medical devices according to the present invention have a surface of which is modified such that Teflon-like structures having -CF, -CF₂, - CF₃ groups or mixtures thereof are covalently bonded to the surface, or branched and/or crosslinked macromolecular networks based on CF, CF₂ or CF₃ units are formed on the surface of the device.

In another aspect, the present invention is directed to a method of modifying the surface of a medical device. In one embodiment, the method includes the step of treating the surface of a medical device wherein at least a portion of the medical device is formed from a polymeric material, with a gaseous plasma to form low energy fluorinated surfaces wherein -CFy groups are covalently bonded to the surface, and wherein y = 1-3, and most desirably, y = 3.

The surface modification may be accomplished by contacting the surface of the medical device with plasma gases which have available fluoride ions and radicals.

For some polymeric surfaces, a surface oxidation treatment may be conducted prior to fluorination, or simultaneously with fluorination. Thus, the present invention may be employed to modify inert polymeric surfaces, such as those surfaces formed from polymeric compositions comprising polyolefins such as polyethylene and polypropylene, and can be employed to modify polymeric surfaces comprising polymers having functionality, such as those polymers having groups which include at least one oxygen, such as hydroxyl, carbonyl, amide, ester, acid, ether, or which have hydrogens next to such groups.

The resultant medical devices having the surface modification described herein include, but are not limited to, catheter assemblies for angioplasty, urological procedures, for use in the biliary duct, for neurological procedures, for use in the reproductive system, for delivery of medical devices such as stents, guide catheters, etc. Specific components of such medical devices having the surface modification herein include, but are not limited to expandable balloons, catheter shafts, guide wires.

The modified surface reduces the frictional resistance of the material, thus reducing sliding resistance, and exhibits improved durability under sliding conditions. The benefits resulting from the modified surface of the device may be obtained without substantially impacting the device dimensions or the bulk properties of the material from which the device is formed. Thus, the modification does not have a substantial negative impact on tensile strength, flexibility or distension properties of the coated material.

Thus, the resultant medical devices exhibit improved characteristics such as reduced frictional resistance, improved durability, higher abrasion and puncture resistance, while the bulk properties of the polymeric composition remain substantially unchanged.

Other aspects of the invention are described in the Detailed Description and in the claims below.

### BRIEF DESCIPTION OF THE DRAWINGS

FIG. 1 is a longitudinal cross-sectional view of a balloon catheter having an inner surface of an inner catheter shaft modified according to the invention.
FIG. 2 is a schematic diagrammatic view of a plasma processing system utilizing a radio frequency (RF) plasma source.
FIG. 3 schematically illustrates a plasma reaction chamber that may be employed for continuous or semi-continuous cold plasma treatment of wire, tubing, and the like.
FIG. 4 illustrates a reaction chamber design that allows for rotation of the substrate and/or for plasma treatment of an internal lumen surface.
FIG. 5 illustrates an alternative arrangement of electrodes for a plasma reaction chamber employed in the invention.
FIG. 6 is a longitudinal cross-sectional view of a balloon catheter having several surfaces that may be modified in accordance with the invention.
FIG. 7 is an ESCA showing an example of the level of carbon and oxygen of a polymer substrate prior to surface treatment.
FIG. 8 is an ESCA showing an example of the surface level of fluorine which may be achieved after surface treatment.

### DETAILED DESCRIPTIONS OF THE PREFERRED EMBODIMENTS

While this invention may be embodied in many different forms, there are described in detail herein specific embodiments of the invention. This description is an exemplification of the principles of the invention and is not intended to limit the invention to the particular embodiments illustrated. The present invention relates to medical devices having a surface modified with covalently bonded fluorine. In specific embodiments, the portion of the medical device whose surface is modified with fluorine, is formed from a polymeric composition.

While the surface of any medical device may be modified using the techniques described herein, the present invention finds utility for catheter assemblies. Catheter assemblies are employed in a wide range of procedures and are used for example, for procedures in the vasculature including the coronary and peripheral vasculature, in the biliary duct, in the neurological system, in the urinary tract, in the reproductive system, as well as guide catheters and delivery systems for medical devices such as stent delivery systems. The present invention may be employed to modify the catheter shafts as well as the surfaces of expandable balloon members for the catheter assemblies used in these procedures.

The present invention is useful for modification of the inner surfaces of the catheter assembly or the inner surfaces of components of the catheter assembly such as the inner surface of an inner or outer catheter shaft, or the inner surface of an expandable member.

The present invention may also be employed to modify other surfaces such as guide wire surfaces, inner and outer surfaces of stent retaining sleeves.

These are only examples of the types of medical devices for which the present invention may be employed, and is not intended to limit the scope of the present invention.

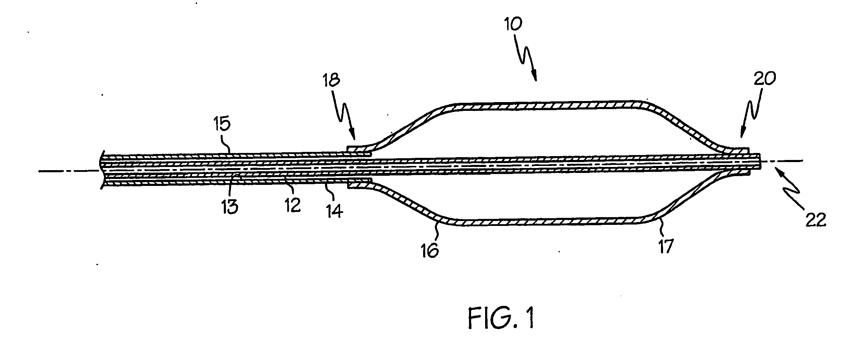

In one embodiment, the present invention is directed to a catheter shaft having the inner surface of the catheter shaft modified according to the invention to reduce the wire movement friction when a guide wire is inserted therethrough. FIG. 1 is a longitudinal cross-sectional view of the distal end of a conventional balloon catheter 10 having an inner shaft 12, an outer shaft 14 and a expandable balloon member 16 bonded to the outer shaft 14 at the proximal end 18 and to the inner shaft 10 at the distal end 18. Optionally, the balloon catheter 10 could be provided with a distal tip (not shown) to which the distal end 20 of balloon 16 is bonded. Bonding can be accomplished through any conventional means including, for example, welding or adhesive bonding. Inner shaft 12 having an inner surface 13 is shown with a guide wire 22 extending therethrough. The inner surface 13 of inner shaft 12 may be modified according to the invention to reduce the coefficient of friction between the inner surface 13 of inner shaft 12 and the guide wire 22 and thus to improve the wire movement therethrough. Further, the outer surface 15 of outer shaft 14 and the outer surface 17 of balloon 16 may also be modified according to the invention to improve the lubricity thereof.

Surface modification includes contacting the surface of the device with plasma gases suitable for modifying the surface such that Teflon-like structures having CF, CF₂, CF₃ groups on the surface, or mixtures thereof such groups, or having branched and/or crosslinked macromolecular networks based on CF, CF₂ or CF₃ units.

The plasmas employed herein are those having available fluoride ions and radicals. As used herein, the term "fluorine" shall hereinafter refer to both those atoms which are charged (fluoride ions) and neutral species.

The fluorination process involves abstraction of oxygen and/or hydrogen from the surface and groups of-CF_{y} where y = 1-3, are formed. Exemplary groups on the surface of the device include, but are not limited to, ether (RC-O-CR), acid (-COOH), ester (-COR), amide (-CONH₂), hydroxyl (-OH), carbonyl (-C=O) or hydrogens (H) adjacent said groups prior to conversion of these groups to -CF_{y} where y =1-3 or prior to formation of branched and/or crosslinked macromolecular networks based on -CF, -CF₂ or -CF₃ units.

Teflon-like structures may also be formed on the surface of the device using cyclic aromatic fluorocarbons such as hexafluorobenzene and perfluorinated cyclic fluorocarbons such as dodecafluorocyclohexane, octadecafluorodecalin. These compounds undergo ring-opening processes under cold plasma conditions, rather than defluorination. These structures can produce high-fluorine-content macromolecular layers. Plasma-generated layers having branched and/or crosslinked macromolecular networks based on CF, CF₂, and CF₃ units may be formed using these compounds.

The modification by plasma treatment occurs on the surface of a substrate, and thus, does not substantially change bulk polymer properties. Covalent bonds are broken and reformed on the surface such that modification may occur only in the first 10-10,000 Ångstroms, more suitably 10 to 1000 Angstroms and even more suitably 10 to 100 Ångstroms.

Any polymeric composition may be employed in this invention. If the polymeric material is of a more inert character, such as polyolefin materials, wherein little or no available oxygen is present on the surface in the form of functional groups as mentioned above, then atomic oxygen may be incorporated into the process in order to first oxidize the surface of the medical device.

The medical device may be formed from both elastomeric and non-elastomeric polymeric materials, and even relatively inert polymeric surfaces such as those which have carbon-hydrogen bonds, for example, polyolefinic surfaces including those formed with polyethylene or polypropylene, may be fluorinated using the method of the present invention.

While non-aromatic materials are more preferable for use herein due to the ease of abstracting hydrogens from such surfaces, those having aromaticity, are also suitable for use herein, providing the appropriate functional groups are present for hydrogen and/or oxygen abstraction.

Examples of polymeric materials suitable for use herein include, but are not limited to, silicone resins, phenolic resins, polyolefins, polyvinyls, polyesters, polyacrylates, polyethers, polyketones, polyamides including the nylons, polysulfones, cellulosic materials, polystyrene, polyisobutylene, polybutene, polyurethanes, polycarbonates, polyepoxides, polyacrylonitriles (PAN), poly(meth)acrylates, block copolymers, copolymers thereof, and mixtures thereof, as well as a wide variety of other polymeric materials not specifically mentioned herein. As used herein, the term "copolymer" shall be used to refer to any polymer formed using two or more monomers including terpolymers.

Examples of suitable polyolefins include polyethylene, polypropylene as well as copolymers thereof.

Polyamides including any of the nylons such as nylon 6, nylon 6/6, nylon 6/12, nylon 9/12, nylon 6/10, nylon 10, nylon 11, nylon 12, etc., may be employed herein.

Examples of suitable polyamide block copolymers include, for example, the poly(ether-block-amides) such as those available under the tradename of PEBAX® available from Atofina Chemicals in Philadelphia, PA.

Examples of suitable polyesters include, but are not limited to, polyethylene terephthalate (PET), polybutylene terephthalate (PBT), poly(ethylene 2,6-naphthalate) (PEN). Examples of polyester block copolymers include, but are not limited to, polyester-block-ester copolymers, polyester-block-ether copolymers Poly(ester-block-ether) elastomers are available under the tradename of HYTREL® from DuPont de Nemours & Co. and consist of hard segments of polybutylene terephthalate and soft segments based on long chain polyether glycols. These polymers are also available from DSM Engineering Plastics under the tradename of ARNITEL®.

Examples of polyether copolymers include polyetheretherketones (PEEK).

Examples of suitable styrenic block copolymers include, but are not limited to, those block copolymers having styrenic endblocks, including, but not limited to, styrene-isoprene-styrene (SIS), styrene-butadiene-styrene (SBS), styrene-ethylene/propylene-styrene (SEPS), styrene-isobutylene-styrene (SIBS), styrene-ethylene/butylene-styrene (SEBS).

Block copolymer elastomers employed in balloons, for example, are described in commonly assigned US Patent Nos. 6406457, 6171278, 6146356, 5951941, 5830182, 5556383, 5112900.

Examples of commonly used polymeric materials for forming medical balloons include, but are not limited to, polyesters, polyamides, polyolefins, copolymers thereof, and mixtures thereof. Suitable balloon materials are described in commonly assigned U.S. Patent Nos. 5549552, 5447497, 5348538,5550180,5403340,6328925.

Specific examples of polymeric materials suitable for forming catheter shafts include, but are not limited to, polyolefins such as polyethylene, polyethylene terephthalate, polybutylene terephthalate, poly(ether-block-amide), poly(ester-block-ether), poly(ester-block-ester).

In one specific embodiment, the medical device is a catheter balloon assembly used for coronary angioplasty, and the polymeric material from which the balloon and/or inner shaft is formed is a polyether-block-amide, such as those available under the tradename of PEBAX® from Atofina in Philadelphica, PA or nylon, PET, polyethylene, or combinations thereof.

Of course, multilayer structures may also be employed herein where two or more polymer layers are formed using different polymeric compositions. The same polymeric composition may also be employed as an alternating layer, for example.

Catheters may be formed of conventional materials of constructions that are described in detail in the art. The proximal shaft section can be manufactured by multi-lumen extrusion using a high-strength polymer such as a polyolefin, polyalkylene terephthalate, nylon, poly(ether-block-amide), polyetheretherketone (PEEK). Coextrusion can be employed to form a multilayer structure as well.

For some catheter construction, a first polymeric composition is employed to form the outer shaft, and a second polymeric composition is employed to form an expandable member, and the two are bonded together adhesively, or by welding, for example. The expandable member may then be connected at the distal end, to an inner shaft. The surface of the inner shaft may be modified according to the invention.

Other optional components may be incorporated into the polymeric composition including, but not limited to, micro and nano particulate materials having diameters of 10 microns or less, fibrous materials, fillers, antioxidants, plasticizers, waxes, biocides, crosslinkers, heat stabilizers, therapeutics. Such optional components are known to those of skill in the art.

Fibrous material in the form of braiding, weaving, knitting, roving, random, etc. may be provided within a layer, or between layers as well.

In one embodiment, the present method is used to form multilayer catheter tubing in which at least one layer is a nano-composite material. The nano-composite material may be employed as an inner, intermediate, or an outer layer.

Polymer nanocomposites (nanotubes, nanoparticles, nanoclays; nanospheres) are a relatively new class of composites which are a blend of nanometer-sized (10⁻⁹ meter) fillers with either a thermoset or thermoplastic polymer. Due to the size of the dispersed particles, the nanocomposites exhibit modified mechanical, thermal and optical properties as compared to pure polymers or conventional composites. Commonly, nanocomposites are based on clays and layered silicates. Other examples include carbon nanotubes and ceramic nanoparticels.

Commercially available nano-composite materials include, but are not limited to, nano-clays available from Nanocor, Inc. of Arlington Heights, III. under the trademark NANOMER® and from Southern Clay Products, Inc. of Gonzales, Tex. under the trademark CLOSITE®.

Nanocomposite materials are discussed in US Patent Nos. 6770697 and 6770696, for example.

In one embodiment, a trilayer structure is formed by incorporating a nano-composite material, such as SEEP® nanocomposite of nylon 12 and <10% nanometer size clay particles available from Foster Corp. in Dayville, CT, between an outer polymeric layer and an inner polymeric layer, the inner and/or outer layer modified using the surface modification techniques according to the invention.

The above lists are intended for illustrative purposes only, and not as a limitation on the scope of the present invention.

The surfaces of the medical devices constructed of the polymeric compositions described above, may be modified using fluorine-containing gaseous plasmas as described below.

Fluorination may be accomplished using gaseous plasmas having available fluorine atoms or fluoride ions, i.e. neutral and charged species as well as radicals. Any suitable gaseous source of charged and neutral species of fluorine atoms including radicals may be employed herein. The fluorine-containing compounds are dissociated during the plasma process to form fluorine atoms in the form of charged and neutral species. Examples of such gaseous compounds include, but are not limited to, fluorine gas, gases comprising C*ₓ*F*_{y}* wherein x =1-5 and *y* = 3-8 including, but not limited to, CF₃, CF₄, C₂F₄, C₂F₆, C₃F₇, C₃F₈, C₄F₈, C₄F₆, C₅F₈, etc. Within this group are those having the general formula (CF₂)ₓ where x =1-4. Other suitable gases comprise SF₆ (sulfur hexafluoride), HF, NF₃, PF₅, CₓF_{y}X_{z} (X = H, Cl, Br) such as CHF₃, CH₃F CH₂F₂, CHClF₂, CCl₂F₂, CCl₃F, etc. wherein x = 1, y =1-3, z = 1-3 and y + z = 4.

Of course, some fluorine containing compounds are of course more preferable for use than others due to expense, and relative ease of dissociation, for example. Other considerations relating to the application of the final product, as well as manufacturing equipment, may also be a factor in the selection of the plasma gas. One of skill in the art is knowledgeable of such plasmas.

As discussed briefly above, fluoropolymers may also be employed in the plasma processes according to the invention. For example, cyclic aromatic fluorocarbons and perfluorinated cyclic fluorocarbons, for example, hexafluorobenzene, dodecafluorocyclohexane, octadecafluorodecalin, are known to undergo ring-opening processes under cold plasma conditions. Using these types of compounds, fluorocarbon groups having the general formula -CₓF_{y} are deposited on the surface wherein x =1-5 and y = 4-9 including -CF₃, rather than fluorine atoms. This type of process is described in *Synthesis and Characterization of Teflon-Like Macromolecular Structures from Dodecafluorocyclohexane and Octadecafluorodecalin Under RF-Cold-Plasma Conditions,* F. Denes et al., Journal of Applied Polymer Science, Vol. 71,1627-1639, John Wiley & Sons, Inc., 1999. Cold plasma reactors are also described in U.S. Patent No. 6,096,564.

In one embodiment, the plasma employed contains SF*ₓ* where *x* ≤ 6.

The above lists of gaseous plasmas are intended for illustrative purposes only, and do not limit the scope of the present invention. Such compounds are known to those of ordinary skill in the art for use in plasma treatments.

It may be advantageous, although not a requirement, to employ, in combination with the reactive gases employed herein, at least one inert gas. It has been found that the use of an inert gas, such as a noble gas, may facilitate desirable reactants, due to the metastable energies of the noble gas(es) that are available thus resulting in ap referred chemical species and bonding states at the substrate surface. For example, inert gases such as argon (Ar) and helium (He) may be employed in combination with nitrogen (N₂), hydrogen (H₂), oxygen, etc. as well as the gases comprising the source of fluorine, whereby through this process, fluorine atoms become bonded to the polymer surface at the molecular level. The term "inert" as used herein is indicative of relative chemical inactivity of the gas or polymer to which it herein refers.

Such techniques are known to those of skill in the art.

The inert gas may be employed in any suitable amount. However, such gases are often employed in amounts of about 25% or more, and more suitably about 50% or more by volume. For example, in some embodiments, inert gas(es) may be employed in an amount of composition, by volume, of 70 to 95% of inert gas and 30 to 5% of gas of a fluorine-containing compound such as SF₆. These compositions are intended for illustrative purposes only, and not as a limitation on the scope of the present invention. These ranges are exemplary only, and not intended to limit the scope of the present invention.

In one embodiment, a mixture of Ar/O₂ is employed. These gases may be used in sequentially with the fluorination step whereby the substrate is first exposed to the Ar/O₂ mixture, or the gases may be used simultaneously. For example, the Ar/O₂ gas and the fluorine-containing gas may be fed into a mixing chamber prior to exposing the substrate to the mixture.

As noted above, for materials having little or no available oxygen on the surface, oxygen may be incorporated into the process to oxidize the polymeric material and to produce oxygen-containing groups on the surface. Thus, for example, fluorination of polyolefinic materials such as polyethylene, oxygen may be incorporated into the process. The oxidation step may also be accomplished sequentially, prior to the fluorination treatment, or it may be accomplished simultaneously with one of the steps above by mixing oxygen therein, or all gases may be mixed in the mixing chamber prior to exposing the substrate to the gas(es).

Plasma generation is known in the art, and any known methods may be used herein. For example, US Patent Nos. 5521351, 6083355, 6106659, describe different methods of plasma generation.

A typical method may involve the steps of inserting an article into a treatment chamber that can be sealed and is substantially gastight, passing into the treatment chamber a basic gas mixture comprising a fluorine-containing chemical compound capable of being converted to elemental fluorine, splitting the fluorine-containing compound with an RF-glow discharge plasma to produce a gas containing elemental fluorine, and fluorinating the article. It is often beneficial to employ an inert gas with the fluorine-containing chemical as is explained further below.

A variety of plasma processing techniques and sources of generating plasma are available including microwave, electron cyclotron resonance (ECR), microwave coupled with ECR, direct current (DC), RF-glow discharge, inductively coupled plasmas or helicon wave generators.

A typical plasma processing system generally, may include a variable pressure reaction chamber, a power supply, a vapor- and gas-feeding system (monomer and gas reservoirs, flow controllers, valves), and a vacuum installation (mechanical vacuum pump, valves, liquid nitrogen trap and *in situ* and *ex situ* connecting lines.

Desirably, the present invention is conducted at atmospheric pressure. The reaction chamber further includes at least two electrodes.

The gas may be passed through a reaction zone and exposed to therein to a radio frequency excitation, microwave excitation, electrodes. The discharge, regardless of which type is employed, i.e. glow, corona, arcing, is maintained at a sufficiently high energy to form desired fluorine ions or radicals. Corona discharge and arc discharge are typically employed for other applications than reactive chemistry. For example, arc discharge may be used for cutting, welding and plasma spraying, for example.

For polymeric substrates, cold plasma processes at atmospheric pressure are desirable as polymeric substrates can be damaged by high temperatures.

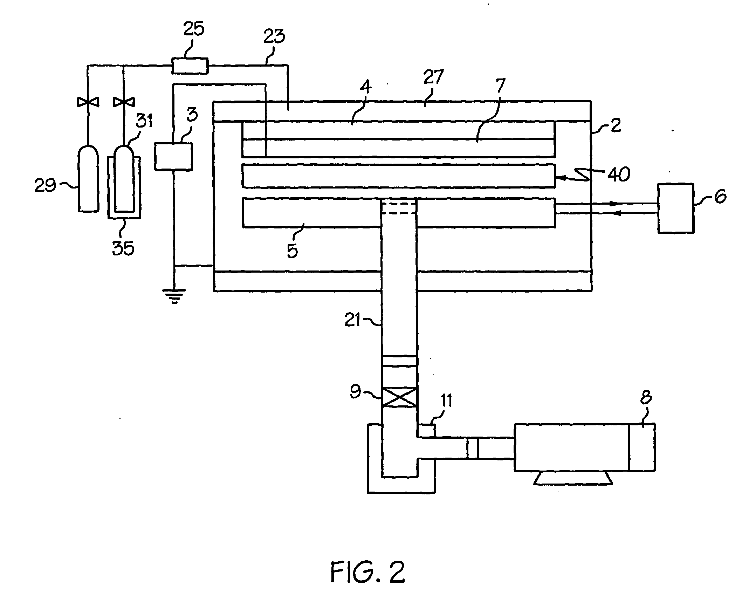

One configuration of a RF-glow discharge system which may be employed herein is shown as a simplified schematic view in FIG. 2.

A drum-type stainless steel electrode 1 is positioned in cylindrical reaction chamber 2 which also may be formed from stainless steel. The electrode 1 is connected to the RF-power supply 3. An electric insulator disc 4 assures insulation of the upper electrode 1 from top of the reactor, which is commonly stainless steel. An electrically heated lower electrode 5 is connected to a temperature controller 6. Electrically heated lower electrode 5 is part of the vacuum line 7 which is connected to a mechanical vacuum pump 8 for evacuation of the chamber. Evacuation of the chamber 2 is performed through the gap existing between the lower electrode and the bottom of the reaction chamber which is equipped with a valve 9. A liquid nitrogen trap 11 is located below for capture of condensates.

The substrate for which the surface is to be modified, in this embodiment catheter tube 3, is placed adjacent the electrode 1.

Gases containing a source of fluoride ions and radicals may be introduced into the reaction chamber 2 through gas inlet 23 coupled with a flow controller 25 where they are dissociated by RF plasma. Shown in this embodiment is a gas mixing chamber 27 wherein the desired gases may be mixed prior to entry into the reaction chamber 2 through the use of mixing control valves and flow rate control. Inert gases such as He or Ar may be mixed with the fluorine-containing gas in mixing chamber 27 and optionally O₂ if so required. In one embodiment, a gaseous Ar/H₂ blend, the fluorine-containing gas, and optionally oxygen, if needed, may be mixed therein. Gases may be supplied from one or more gas tanks 29, 31 which may then be supplied to the mixing chamber 27 via inlet 23. Tank 31 is shown optionally equipped with a heated reservoir 35. The use of a heated reservoir can reduce the voltage required for ionization, and some reactants require volatilization before they can be employed.

Cold plasma reactor configurations are known in the art. Various modifications may be made to such reactors which are within the purview of those of ordinary skill in the art.

Radical fluorine species with sufficient lifetime reach the substrate where surface modification may occur. In this embodiment, substrate 40, which may be a catheter tube, is shown adjacent upper electrode 1.

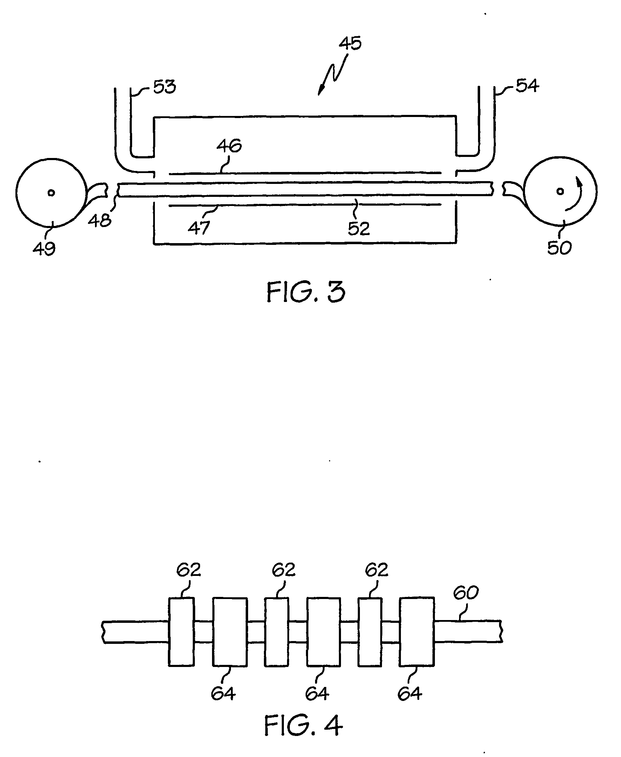

FIG. 3 illustrates another embodiment of a reaction chamber 40 adapted of continuous or semi-continuous deposition on a tubular or wire-like substrate. The reaction chamber 45 employs upper and lower electrodes, 46, 47 respectively, that may be biased by an RF source in a manner similar to that of the electrodes in FIG. 2. A tubular or wire-like substrate 48, such as catheter tubing or guide wire is moved by a suitable motive source from reel 49 to reel 50 during the treatment process, passing through the gap 52 between the electrodes. The appropriate gas(es) are provided to, and removed from, the reactor via ports 53 and 54. In at least some embodiments the chamber is configured so that the flow of gas through the chamber is in the opposite direction of the movement of the substrate 48 through the chamber.

The reaction chamber 45 is suitably operated under vacuum at ambient or near ambient temperature, e.g. 10 - 50°C. The chamber 45 may be enclosed within a larger reactor housing, not shown, that also encompasses reels 49 and 50 at below ambient pressure.

When gas is flowed into the reaction chamber 45 and the RF source is activated, a plasma is generated in the gap resulting in fluorine generation and fluorination of the surface.

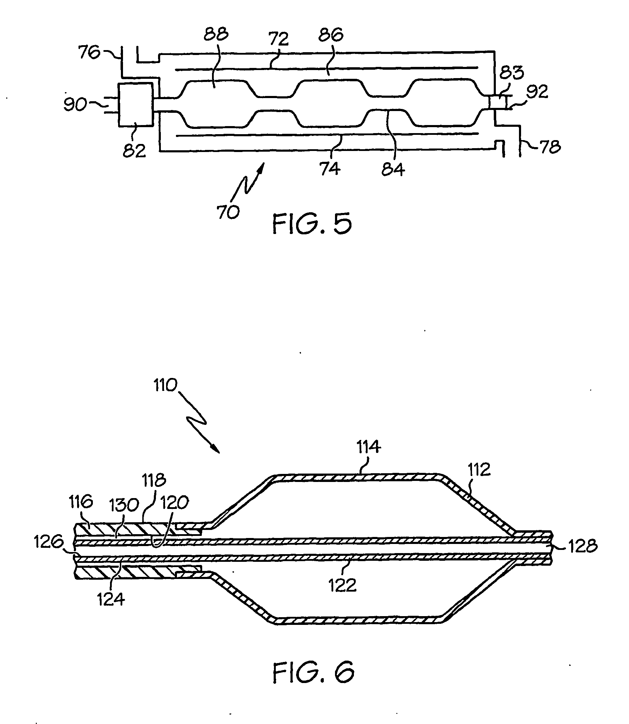

FIG. 4 depicts a further variation of a reaction chamber. The chamber 70 is provided with upper and lower electrodes 72, 74, respectively, suitably powered and insulated as in Fig. 1. Gas flows in one of the ports 76, 78 and out the other. A motor 82 and bearing structure 83 allows rotation of the substrate 84. In the particular case of FIG. 5, the substrate 84 is a series of balloons that are all blown from a single parison. Such balloons suitably are separated by cutting the parison after the plasma treatment. Alternatively separate balloons may be daisy-chained together to allow for multiple single balloons to be processed concurrently. Gas(es) may be flowed into the gap 84 between the substrate and the electrodes, or into the internal substrate volume 88, or both. Gas(es) may be flowed into gap 84 and an inert gas flowed into volume 88, or visa versa. Different plasma gases may be flowed on the outside and inside of the substrate.

FIG. 6 illustrates a distal segment of a balloon catheter 110 that includes a balloon 112 having an outer surface 114. Catheter 110 also includes an outer shaft 116 having outer and inner surfaces 118, 120, respectively, and in inner shaft 122 having outer and inner surfaces 124, 126, respectively. The inner shaft defines a guide wire lumen 128. The space between the inner and outer shafts defines an inflation lumen 130. The balloon 112 is bonded on its proximal end to the outer shaft 116 and on its distal side to the inner shaft 122.

Sliding surfaces of the catheter 110 include at least the inner surface 126 of the inner shaft 122, the outer surface 118 of the outer shaft 116, and the balloon outer surface 114. The inner surface 126 slides over a guide wire during deployment. Outer shaft surface 118 and a portion of the outer balloon surface 114 slide thorough the body vessel, for deployment and removal. In some cases the inner and outer shafts are made movable relative to each other so there may be sliding of inner shaft surface 126 relative to outer shaft surface 120.

To facilitate coating of inner surfaces, the plasma generating gas may be fed through the substrate as well as around it. In some embodiments of the invention a tubular substrate is provided and the plasma generating gas is fed only through the interior of the device, not around the outside, so the plasma is not generated on the outside of the tube. In other embodiments the interior of the device is sealed, or is separately fed with neutral gas that does not generate plasma, while plasma generating gas is fed around the outside of the substrate. In such cases the coating is provided only on the outside surface of the device. In still another variation, different plasma generating gases may be provided to the interior of the device and the outside. For instance, plasma gas(es) may be provided to the interior and/or exterior of the device. Furthermore, different plasma gas(es) may be provided concurrently with the fluorine source.

Fluorination of the device can produce a surface which has low contact adhesion and thus low sliding resistance. Fluorination occurs at the surface level, and thus causes little or no change in the bulk properties of the polymeric material upon which it is deposited.

Fluorination of polymers using plasma discharge methods are discussed in, for example, US Patent Nos. 4902529, 4491653, 4296151, 4264750, 4020223.

Desirably, the plasma processing method employed is one in which a high concentration of -CF₃ groups are covalently bonded at the surface wherein the layer modified is between about 10 and 10,000 Å, more suitably about 10 to about 1000 Å and most suitably about 10 to about 100 Å.

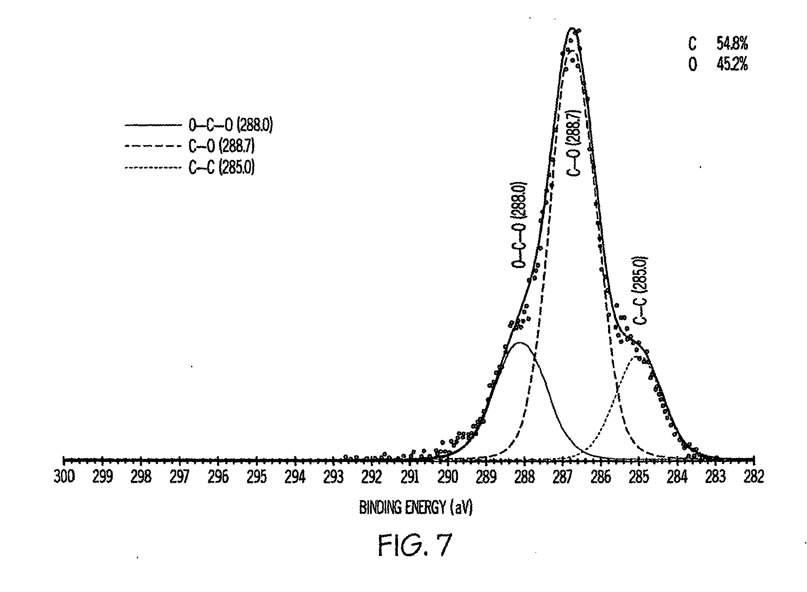

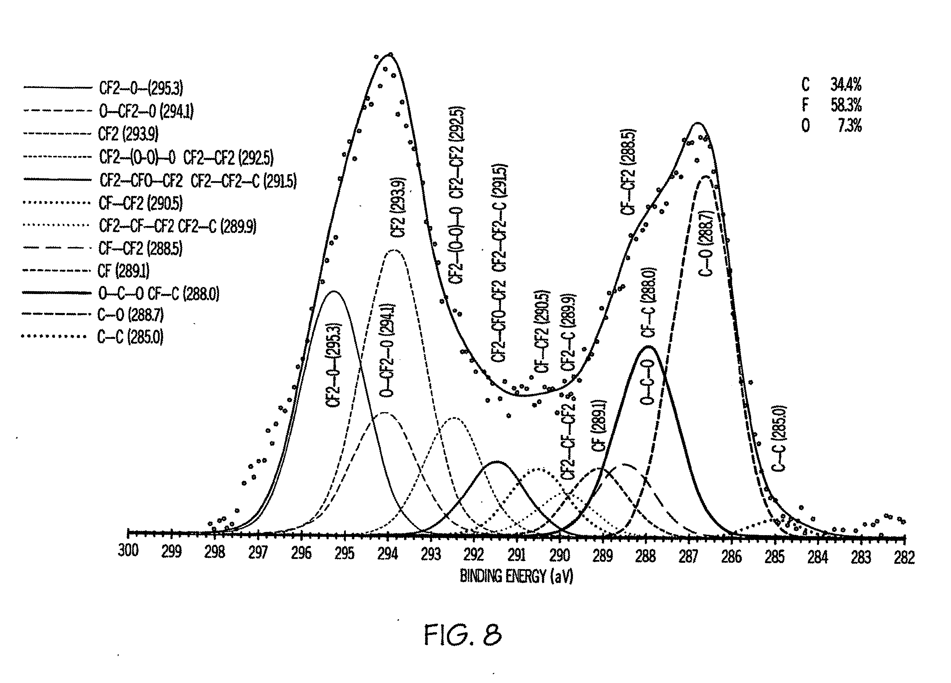

FIGS. 7 and 8 is an ESCA graph illustrate a level of fluorination which may be achieved using a fluorination process of the type described herein. FIG. 7 shows

Reactor conditions may be set according to the depth of surface modification desired, the type of gases employed, the type of polymer composition employed in formation of the substrate to be modified.

Conditions such as substrate temperature, chamber pressure, frequency and level of electrical excitation and gas flow rate(s) may determine the composition and properties of the deposited layer. Using routine experimentation, one of ordinary skill in the art could adjust these factors in order to achieve the desired results.

For many polymeric compositions, it is desirable to avoid high temperatures which would degrade the surface of the polymer being treated. Thus, a cold plasma system may be desirably employed.

Reactor conditions for cold plasma reactions, for example, may involve RF power dissipated to the electrodes of about 10 to about 10³ W, power density of about 0.1 to about 10 W/cm², base pressure in the reactor about 20 to 50 Torr, gas flow rate about 0.01 to about 5 cm³/minute, and temperature of the reactor at about 10°C to about 120°C may suitably be employed, suitably about 20°C to about 70°C with ambient temperature being desirable. For medical devices or components thereof which are formed from polyamide or polyalkyleneterephthalate, a temperature of about 20° C to about 50°C is desirable.

In one embodiment, a reactor temperature of about 70°C to about 75°C was utilized in combination with an SF₆ containing gas.

For surface treatment of expandable medical balloons, it may be desirable to employ temperatures at the lower end of the temperature range.

Selective fluorination of the surface of the article being fluorinated may be controlled by placement of the electrodes wherein only the desired area of the article to be fluorinated is located between the electrodes. Obviously, more than one set of electrodes may be employed and multiple areas selectively fluorinated as well.

The present invention can be advantageously employed for treatment of inner surfaces of medical devices such as the inner surface of a tubular member, by creating the gaseous plasma inside of the tubular member.

It is important to note that prior to fluorination of the article, the chamber is purged with an inert gas that is substantially pure in order to remove contaminants from the reaction chamber. These purging processes are often accomplished at elevated temperatures as well.

The degree of surface modification may be determined by various well known surface analysis techniques including, but not limited to, Attenuated Total internal Reflectance infrared spectroscopy (ATR IR), Electron Scattering for Chemical Analysis (ESCA), contact angle measurements, atomic force microscopy (AFM) and scanning electron microscopy (SEM).

FIG. 7 is an ESCA showing the surface level of carbon and oxygen of a polymer substrate formed from poly(ether-block-amide) block copolymer, note the absence of fluorine, prior to plasma treatment.

FIG. 8 is an ESCA showing the increased level of fluorine on the polymer surface shown in FIG. 3 after treatment using SF₆ according to the present invention. As can be seen from FIG. 8, a significant amount of oxygen atoms can be replaced with fluorine atoms employing a fluorination process according to the invention. Fluorine atoms have also replaced some hydrogen atoms as well as shown by this ESCA graph.

The present invention may also be employed in combination with other plasma reactions to attach additional functional groups to the surface such as high molecular weight monomers, acrylate or epoxide oligomers.

The present invention allows for maintenance of the bulk properties of the polymer substrate, while modifying surface characteristics only.

The above disclosure is intended to be illustrative and not exhaustive. This description will suggest many variations and alternatives to one of ordinary skill in this art. All these alternatives and variations are intended to be included within the scope of the attached claims.

## Claims

1. A process for modifying the surface of a catheter assembly or a component thereof, at least a portion of which comprises a polymeric composition, the process comprising exposing said surface of said medical device to gaseous plasma sufficient to covalently bond fluorine to said surface of said medical device, the gaseous plasma having available fluoride ions and fluorine radicals.

2. The process of claim 1, the process comprising exposing said surface of said medical device to gaseous plasma comprising available fluorine atoms to form -CF_{y} groups wherein y = 1 to 3, on said surface of said medical device.

3. The process of claim 1 wherein said surface of said medical device comprises functional groups which are selected from the group consisting of ether, acid, ester, amide, hydroxyl, carbonyl, hydrogens (H) adjacent said functional groups, or a combination thereof.

4. The process of claim 1 wherein said surface of said medical device is subjected to a surface oxidation treatment.

5. The process of claim 4 wherein said surface oxidation treatment is simultaneously conducted with exposing said surface to said gaseous plasma comprising available fluorine atoms.

6. The process of claim 1 wherein said polymeric composition comprises at least one member selected from the group consisting of polyolefins, polyesters, polyamides, copolymers thereof and mixtures thereof.

7. The process of claim 6 wherein said polymeric composition is polyethylene, polyamide-block-ether, or mixtures thereof.

8. The process of claim 1 wherein said gaseous plasma comprises a source of available fluorine atoms, said source selected from the group consisting of SF₆, CₓF_{y} where x = 1-5 and y = 3-8, PF₅,NF₃, CₓF_{y}X_{z} where X = H, Cl, Br and x = 1, y = 1 -3, z = 1-3, and y + z = 3 or 4, and mixtures thereof.

9. The process of claim 1 wherein said plasma comprises CF_{y} where y < 4 or SFₓ where x ≤ 6.

10. The process of claim 1 wherein said medical device is a catheter assembly comprising a catheter shaft having an inner surface and an outer surface, at least a portion of the inner surface exposed to said plasma.

11. The process of claim 3 wherein a majority of said groups are converted to -CF₂ and -CF₃.

12. The process of claim 3 wherein a majority of said groups are converted to - CF₃.

13. The process of claim 1 wherein said process is a cold plasma process.

14. The process of claim 1, said process further comprising the step of extruding said polymeric composition to form a tubular member prior to modifying said surface of said polymeric composition.

15. The process of claim 14, said process further comprising the step of molding said tubular member to form a medical balloon.

16. The process of claim 1 in combination with a second surface modification process.

17. The process of claim 14 wherein said tubular member has an inner surface and an outer surface, and said process comprising the step of modifying at least the inner surface of said tubular member.

18. A medical device obtainable according to the process of any of claims 1-17, the device comprising a tubular member having an inner surface and an outer surface, at least a portion of said inner surface of said tubular member having fluorine atoms covalently bonded thereto.

19. The medical device of claim 18, wherein said tubular member is formed from a polymeric composition comprising a non-fluoropolymeric material, and said fluorine atoms are covalently bonded to said surface of said tubular member represented by the formula -CFₙ wherein n is 1, 2, or 3.

20. The medical device of claim 19 wherein a majority of fluorine atoms are covalently bonded to said surface of said tubular member represented by the formula -CFₙ wherein n is 2 or 3.

21. The medical device of claim 19 wherein said medical device is a catheter assembly and said tubular member is a catheter shaft.

22. The medical device of claim 19 wherein said polymeric composition comprises at least one member selected from the group consisting of polyesters, polyethers, polyamides, polyimides, poryolefins, polycarbonates, block copolymers, and mixtures thereof.

23. The medical device of claim 19 wherein aid polymeric composition comprises at least one member selected from the group consisting of polyethylene terephthalate, poly(ether-block-amide), poly(ester-block-ether), poly(ester-block-ester), polyethylene and mixtures thereof.

24. A catheter assembly comprising a shaft, the shaft having a matrix, an inner surface and an outer surface, the shaft formed from a polymeric material comprising at least one oxygen-containing functional group, or hydrogens adjacent to said functional groups, said surface modified by fluorination wherein -CF_{y} groups, where y = 1-3, are formed on at least one of said inner surface or said outer surface or both, but not in the matrix.

25. The catheter of claim 24, said polymeric material comprising functional groups selected from the group consisting of ether, acid, ester, amide, hydroxyl, carbonyl, hydrogens (H) adjacent said functional groups, or a combination thereof.

26. A process for modifying a surface of a catheter assembly, the process comprising the step of exposing said surface of said medical device to a gaseous plasma comprising cyclic aromatic fluorocarbons, perfluorinated cyclic fluorocarbons or mixtures thereof.

27. A catheter assembly or component thereof comprising a surface, that is modified by having fluorine covalently bonded thereto, said surface comprising at least one plasma generated layer, said layer comprising branched or crosslinked macromolecular networks based on CF, CF₂, CF₃ units or mixtures thereof, wherein the at least one plasma generated layer is obtainable using a gaseous plasma having available fluoride ions and fluorine radicals.

## Patentansprüche

1. Verfahren zum Modifizieren der Oberfläche eines Kathetergefüges oder eines Bestandteils davon, wobei zumindest ein Teil davon eine Polymerzusammensetzung umfasst, wobei das Verfahren das Aussetzen der Oberfläche der medizinischen Vorrichtung an zum kovalenten Binden von Fluor an die Oberfläche der medizinischen Vorrichtung ausreichendes gasförmiges Plasma umfasst, wobei das gasförmige Plasma verfügbare Fluoridionen und Fluorradikale aufweist.

2. Verfahren nach Anspruch 1, wobei das Verfahren das Aussetzen der Oberfläche der medizinischen Vorrichtung an gasförmiges Plasma umfasst, das verfügbare Fluoratome zum Bilden von -CF_{y}-Gruppen, wobei y = 1 bis 3, auf der Oberfläche der medizinischen Vorrichtung umfasst.

3. Verfahren nach Anspruch 1, wobei die Oberfläche der medizinischen Vorrichtung funktionelle Gruppen umfasst, die ausgewählt sind aus der Gruppe, bestehend aus Ether, Säure, Ester, Amid, Hydroxy, Carbonyl, Wasserstoffen (H) benachbart zu funktionellen Gruppen oder einer Kombination davon umfasst.

4. Verfahren nach Anspruch 1, wobei die Oberfläche der Oberfläche der medizinischen Vorrichtung einer Oberflächenoxidationsbehandlung unterzogen wird.

5. Verfahren nach Anspruch 4, wobei die Oberflächenoxidationsbehandlung gleichzeitig mit dem Aussetzen der Oberfläche an das verfügbare Fluoratome umfassende gasförmige Plasma umfasst.

6. Verfahren nach Anspruch 1, wobei die Polymerzusammensetzung mindestens einen Vertreter umfasst, der ausgewählt ist aus der Gruppe, bestehend aus Polyolefinen, Polyestern, Polyamiden, Copolymeren davon und Gemischen davon.

7. Verfahren nach Anspruch 6, wobei es sich bei der Polymerzusammensetzung um Polyethylen, Polyamid-Block-Ether oder Gemische davon handelt.

8. Verfahren nach Anspruch 1, wobei das gasförmige Plasma eine Quelle für verfügbare Fluoratome umfasst, wobei die Quelle ausgewählt ist aus der Gruppe, bestehend aus SF₆, CₓF_{y}, wobei x = 1-5 und y = 3-8, PF₅, NF₃, CₓF_{y}X_{z}, wobei X = H, Cl, Br und x = 1, y = 1-3, z = 1-3 und y + z = 3 oder 4, und Gemischen davon.

9. Verfahren nach Anspruch 1, wobei das Plasma CF_{y}, wobei Y<4, oder SFₓ, wobei x≤6, umfasst.

10. Verfahren nach Anspruch 1, wobei die medizinische Vorrichtung ein Kathertergefüge ist, das einen Katheterschaft mit einer inneren Oberfläche und einer äußeren Oberfläche umfasst, wobei zumindest ein Teil der inneren Oberfläche dem Plasma ausgesetzt wird.

11. Verfahren nach Anspruch 3, wobei ein Großteil der Gruppen zu -CF₂⁻ und -CF₃ umgewandelt wird.

12. Verfahren nach Anspruch 3, wobei ein Großteil der Gruppen zu -CF₃ umgewandelt wird.

13. Verfahren nach Anspruch 1, wobei das Verfahren ein Kaltplasmaverfahren ist.

14. Verfahren nach Anspruch 1, wobei das Verfahren des Weiteren den Schritt des Extrudierens der Polymerzusammensetzung zum Bilden eines Röhrenelements vor dem Modifizieren der Oberfläche der Polymerzusammensetzung umfasst.

15. Verfahren nach Anspruch 14, wobei das Verfahren des Weiteren den Schritt des Formens des Röhrenelements zum Bilden eines medizinischen Ballons umfasst.

16. Verfahren nach Anspruch 1 in Kombination mit einem zweiten Oberflächenmodifikationsverfahren.

17. Verfahren nach Anspruch 14, wobei das Röhrenelement eine innere Oberfläche und eine äußere Oberfläche aufweist und das Verfahren den Schritt des Modifizierens zumindest der inneren Oberfläche des Röhrenelements umfasst.

18. Medizinische Vorrichtung, die gemäß dem Verfahren nach einem der Ansprüche 1-17 erhältlich ist, wobei die Vorrichtung ein Röhrenelement mit einer inneren Oberfläche und einer äußeren Oberfläche umfasst, wobei zumindest ein Teil der inneren Oberfläche des Röhrenelements daran kovalent gebundene Fluoratome aufweist.

19. Medizinische Vorrichtung nach Anspruch 18, wobei das Röhrenelement aus einer ein Nicht-Fluorpolymermaterial umfassenden Polymerzusammensetzung gebildet ist und die an die Oberfläche des Röhrenelements kovalent gebundenen Fluoratome durch die Formel -CFₙ dargestellt sind, wobei n 1, 2 oder 3 ist.

20. Medizinische Vorrichtung nach Anspruch 19, wobei ein Großteil der an die Oberfläche des Röhrenelements kovalent gebundenen Fluoratome durch die Formel -CFₙ dargestellt sind, wobei n 2 oder 3 ist.

21. Medizinische Vorrichtung nach Anspruch 19, wobei die medizinische Vorrichtung ein Kathetergefüge und das Röhrenelement ein Katheterschaft ist.

22. Medizinische Vorrichtung nach Anspruch 19, wobei die Polymerzusammensetzung mindestens einen Vertreter umfasst, der ausgewählt ist aus der Gruppe, bestehend aus Polyesterm, Polyethern, Polyamiden, Polyimiden, Polyolefinen, Polycarbonaten, Blockcopolymeren und Gemischen davon.

23. Medizinische Vorrichtung nach Anspruch 19, wobei die Polymerzusammensetzung mindestens einen Vertreter umfasst, der ausgewählt ist aus der Gruppe, bestehend aus Polyethylenterephthalat, Poly(ether-block-amid), Poly(ester-block-ether), Poly(ester-block-ester), Polyethylen und Gemischen davon.

24. Kathethergefüge, umfassend einen Schaft, wobei der Schaft eine Matrix, eine innere Oberfläche und eine äußere Oberfläche aufweist, wobei der Schaft aus einem Polymermaterial gebildet ist, das mindestens eine sauerstoffhaltige funktionelle Gruppe oder Wasserstoffe benachbart zu den funktionellen Gruppen aufweist, wobei die Oberfläche durch Fluorierung modifiziert ist, wobei -CF_{y}-Gruppen, wobei y = 1-3, auf mindestens einer der inneren Oberfläche oder der äußeren Oberfläche oder von beiden, aber nicht auf der Matrix gebildet sind.

25. Katheter nach Anspruch 24, wobei das Polymermaterial funktionelle Gruppen umfasst, die ausgewählt sind aus der Gruppe, bestehend aus Ether, Säure, Ester, Amid, Hydroxy, Carbonyl, Wasserstoffen (H) benachbart zu den funktionellen Gruppen oder einer Kombination davon.

26. Verfahren zum Modifizieren einer Oberfläche eines Kathetergefüges, wobei das Verfahren den Schritt des Aussetzens der Oberfläche der medizinischen Vorrichtung an ein gasförmiges Plasma umfasst, das cyclische aromatische Fluorkohlenstoffe, perfluorierte cyclische Fluorkohlenstoffe oder Gemische davon umfasst.

27. Kathetergefüge oder Bestandteil davon, umfassend eine Oberfläche, die **dadurch** modifiziert ist, dass sie daran kovalent gebundenes Fluor aufweist, wobei die Oberfläche mindestens eine durch Plasma erzeugte Schicht umfasst, wobei die Schicht verzweigte oder vernetzte makromolekulare Netzwerke auf der Basis von CF-, CF₂-, CF₃-Einheiten oder Gemischen davon umfasst, wobei zumindest eine durch Plasma erzeugte Schicht unter Verwendung eines gasförmigen Plasmas mit verfügbaren Fluoridionen und Fluorradikalen erhältlich ist.

## Revendications

1. Un processus pour modifier la surface d'un ensemble de cathéter ou d'un composant de celui-ci, dont au moins une partie comprend une composition polymère, le processus comprenant l'exposition de ladite surface dudit dispositif médical à un plasma gazeux de façon suffisante pour lier de façon covalente du fluor à ladite surface dudit dispositif médical, le plasma gazeux comprenant des ions fluorure et des radicaux fluor disponibles.

2. Le processus de la revendication 1, ce processus comprenant l'exposition de ladite surface dudit dispositif médical à un plasma gazeux comprenant des atomes de fluor disponibles pour former des groupes -CF_{y} avec y = 1 à 3, sur ladite surface dudit dispositif médical.

3. Le processus de la revendication 1, dans lequel ladite surface dudit dispositif médical comprend des groupes fonctionnels qui sont choisis dans le groupe formé par les éthers, les acides, les esters, les amides, les hydroxyles, les carbonyles, des hydrogènes (H) adjacents auxdits groupes fonctionnels, ou une combinaison de ceux-ci.

4. Le processus de la revendication 1, dans lequel ladite surface dudit dispositif médical est soumise à un traitement d'oxydation de surface.

5. Le processus de la revendication 4, dans lequel ledit traitement d'oxydation de surface est effectué simultanément à l'exposition de ladite surface audit plasma gazeux comprenant des atomes fluor disponibles.

6. Le processus de la revendication 1, dans lequel ladite composition polymère comprend au moins un élément choisi dans le groupe formé par les polyoléfines, les polyesters, les polyamides, les copolymères de ceux-ci et les mélanges de ceux-ci.

7. Le processus de la revendication 6, dans lequel ladite composition polymère est le polyéthylène, le polyamide-bloc-éther, ou les mélanges de ceux-ci.

8. Le processus de la revendication 1, dans lequel ledit plasma gazeux comprend une source d'atomes de fluor disponibles, ladite source étant choisie dans le groupe formé par SF₆, CₓF_{y} avec x = 1-5 et y = 3-8, PF₅, NF₃, CₓF_{y}X_{z} avec X = H, Cl, Br et x = 1, y = 1-3, z = 1-3 et y+z = 3 ou 4, et les mélanges de ceux-ci.

9. Le processus de la revendication 1, dans lequel ledit plasma comprend CF_{y}, avec y < 4, ou SFₓ avec x ≤ 6.

10. Le processus de la revendication 1, dans lequel ledit dispositif médical est un ensemble de cathéter comprenant une tige de cathéter ayant une surface intérieure et une surface extérieure, au moins une partie de la surface intérieure étant exposée audit plasma.

11. Le processus de la revendication 3, dans lequel une majorité desdits groupes sont convertis en -CF₂ et -CF₃.

12. Le processus de la revendication 3, dans lequel une majorité desdits groupes est convertie en -CF₃.

13. Le processus de la revendication 1, dans lequel ledit processus est un processus à plasma froid.

14. Le processus de la revendication 1, où ledit processus comprend en outre l'étape d'extrusion de ladite composition polymère pour former un organe tubulaire avant modification de ladite surface de ladite composition polymère.

15. Le processus de la revendication 14, où ledit processus comprend en outre l'étape de moulage dudit organe tubulaire pour former un ballonnet médical.

16. Le processus de la revendication 1 en combinaison avec un second processus de modification de surface.

17. Le processus de la revendication 14, dans lequel ledit organe tubulaire présente une surface intérieure et une surface extérieure, et ledit processus comprend l'étape de modification d'au moins la surface intérieure dudit organe tubulaire.

18. Un dispositif médical pouvant être obtenu selon le processus de l'une des revendications 1 à 17, ce dispositif comprenant un organe tubulaire possédant une surface intérieure et une surface extérieure, au moins une partie de ladite surface intérieure dudit organe tubulaire possédant des atomes de fluor liés de façon covalente à celui-ci.

19. Le dispositif médical de la revendication 18, où ledit organe tubulaire formé à partir d'une composition polymère comprend un matériau non-fluoropolymère, et lesdits atomes de fluor liés de façon covalente à ladite surface dudit organe tubulaire sont représentés par la formule -CFₙ avec n = 1, 2 ou 3.

20. Le dispositif médical de la revendication 19, dans lequel une majorité d'atomes de fluor sont liés de façon covalente à ladite surface dudit organe tubulaire représenté par la formule -CFₙ avec n = 2 ou 3.

21. Le dispositif médical de la revendication 19, dans lequel ledit dispositif médical est un ensemble de cathéter et ledit organe tubulaire est une tige de cathéter.

22. Le dispositif médical de la revendication 19, dans lequel ladite composition polymère comprend au moins un élément choisi dans le groupe formé par les polyesters, les polyéthers, les polyamides, les polyimides, les polyoléfines, les polycarbonates, les copolymères à blocs et les mélanges de ceux-ci.

23. Le dispositif médical de la revendication 19, dans lequel ladite composition polymère comprend au moins un élément choisi dans le groupe formé par le téréphtalate de polyéthylène, le poly(éther-bloc-amide), le poly(ester-bloc-éther), le poly(ester-bloc-ester), le polyéthylène et les mélanges de ceux-ci.

24. Un ensemble de cathéter comprenant une tige, la tige comprenant une matrice, une surface intérieure et une surface extérieure, la tige étant formée d'un matériau polymère comprenant au moins un groupe fonctionnel contenant de l'oxygène, ou des hydrogènes adjacents auxdits groupes fonctionnels, ladite surface étant modifiée par fluoration avec formation de groupes -CF_{y}, avec y = 1-3, sur au moins l'une de ladite surface intérieure ou ladite surface intérieure ou sur les deux, mais non dans la matrice.

25. Le cathéter de la revendication 24, dans lequel ledit matériau polymère comprend des groupes fonctionnels choisis dans le groupe formé par les éthers, les acides, les esters, les amides, les hydroxyles, les carbonyles, des hydrogènes (H) adjacents auxdits groupes fonctionnels, ou une combinaison de ceux-ci.

26. Un processus pour modifier une surface d'un ensemble de cathéter, le processus comprenant l'étape d'exposition de ladite surface dudit dispositif médical à un plasma gazeux comprenant des fluorocarbones aromatiques cycliques, des fluorocarbones cycliques perfluorés ou des mélanges de ceux-ci.

27. Un ensemble de cathéter ou un composant de celui-ci comprenant une surface qui est modifiée en formant sur celle-ci des liaisons covalentes avec du fluor, ladite surface comprenant au moins une couche générée par plasma, ladite couche comprenant des réseaux macromoléculaires réticulés ou ramifiés à base d'unités CF, CF₂ ou CF₃ ou de mélanges de celles-ci, dans lequel la au moins une couche générée par plasma peut être obtenue en utilisant un plasma gazeux comportant des ions fluorure et des radicaux fluor disponibles.
